# EUROPEAN PATENT APPLICATION

(11) **EP 3 816 456 A1**
(43) Date of publication of application: **05.05.2021**
(21) Application number: 19827077.9
(22) Date of filing: 28.05.2019
(51) Int. Cl.: F15B 15/12, A61B 17/28, A61B 34/37

(54) **ROTARY ACTUATOR AND ROBOTIC FORCEPS**

(30) Priority: 26.06.2018 JP 2018120498
(71) Applicant: Kawasaki Jukogyo Kabushiki Kaisha, Hyogo 650-8670 (JP)
(72) Inventor: TANAKA, Hideki, Kobe-shi, Hyogo 650-8670 (JP); ANADA, Tadashi, Kobe-shi, Hyogo 650-8670 (JP); HOMMA, Toshiyuki, Kobe-shi, Hyogo 650-8670 (JP)
(74) Representative: Dehns
(86) International application number: PCT/JP2019/020996
(87) International publication number: WO 2020/003853

(57) **Abstract**

A rotary actuator includes: a housing including an interior space in which a vane is disposed; and a cover that is attached to the housing and covers the interior space. An annular seal groove having a triangular cross-sectional shape is formed between the housing and the cover in a manner to surround the interior space, and an outer sealing member is inserted in the seal groove.

## Description

### Technical Field

The present invention relates to a rotary actuator and robotic forceps including the rotary actuator.

### Background Art

In general, in a rotary actuator driven by a working fluid, a vane is disposed in the interior space of a housing of the rotary actuator, and the interior space of the housing is covered by a cover. For example, Patent Literature 1 discloses a rotary actuator configured such that a sealing member is attached to the vane. The sealing member seals between the distal end surface of the vane and the housing (the housing is referred to as "body tube" in Patent Literature 1), and also seals between the side surface of the vane and the cover.

### Citation List

### Patent Literature

PTL 1: Japanese Laid-Open Patent Application Publication No. 2011-185431

### Summary of Invention

### Technical Problem

In the rotary actuator disclosed in Patent Literature 1, no sealing member is interposed between the housing and the cover. However, in order to prevent the working fluid from leaking from the vane-accommodating space to the outside through between the housing and the cover, it is desirable that a sealing member (e.g., an O-ring) be interposed between the housing and the cover.

In this case, generally speaking, an annular seal groove having a rectangular cross section is formed between the housing and the cover in a manner to surround the interior space of the housing, and an outer sealing member is inserted in the seal groove.

However, in the case of adopting such a seal groove having a rectangular cross section, a gap is formed between the inner side surface of the seal groove and the outer sealing member. Therefore, there is a risk that the working fluid may escape through the gap from one pressure chamber to the other pressure chamber, the one and the other pressure chambers being partitioned off from each other by the vane.

In view of the above, an object of the present invention is to provide a rotary actuator in which an outer sealing member is interposed between the housing and the cover and yet that is capable of hindering the working fluid from escaping from one pressure chamber to the other pressure chamber, the one and the other pressure chambers being partitioned off from each other by the vane. Another object of the present invention is to provide robotic forceps including the rotary actuator.

### Solution to Problem

In order to solve the above-described problems, a rotary actuator according to the present invention includes: a housing including an interior space in which a vane is disposed; and a cover that is attached to the housing and covers the interior space. An annular seal groove having a triangular cross-sectional shape is formed between the housing and the cover in a manner to surround the interior space, and an outer sealing member is inserted in the seal groove.

According to the above configuration, since the seal groove has a triangular cross-sectional shape, the filling ratio of the outer sealing member in the seal groove can be increased compared to a case where the seal groove has a rectangular cross-sectional shape. In this manner, the outer sealing member is interposed between the housing and the cover, and yet the working fluid can be hindered from escaping from one pressure chamber to the other pressure chamber, the one and the other pressure chambers being partitioned off from each other by the vane.

The housing may include: a reference surface positioned around the interior space; and an annular wall surface rising from an outer circumferential edge of the reference surface. The cover may include a protrusion that fits inside the wall surface. An inclined surface may be formed on an outer circumferential edge of a distal end surface of the protrusion, such that the seal groove is formed between the inclined surface and a corner between the reference surface and the wall surface. According to this configuration, in advance of attaching the cover to the housing, the outer sealing member can be disposed at the corner between the reference surface and the wall surface of the housing. This allows the cover to be readily attached to the housing.

For example, a recess that forms a vane-accommodating space together with the interior space may be formed in the protrusion.

The vane may include: a circular pillar whose center is a rotational axis of the rotary actuator; and a plate protruding outward in a radial direction from the circular pillar. The above rotary actuator may further include an inner sealing member attached to the vane, the inner sealing member surrounding the plate and the circular pillar. A notch may be formed in a part of the inner sealing member, the part being positioned on a distal end surface of the plate, at a position corresponding to the reference surface of the housing and the distal end surface of the protrusion. According to this configuration, when fitting the protrusion of the cover to the inside of the wall surface of the housing, the inner sealing member can be prevented from getting caught between the reference surface of the housing and the distal end surface of the protrusion.

The distal end surface of the protrusion may be in contact with the reference surface of the housing. According to this configuration, the amount of leakage from the vane-accommodating space can be reduced compared to a case where the receding surface positioned around the protrusion of the cover is in contact with the top surface positioned around the wall surface of the housing. It should be noted that leakage from the vane-accommodating space to the outside is prevented by the outer sealing member. Therefore, the expression that the amount of leakage from the vane-accommodating space can be reduced herein means that the working fluid can be effectively hindered from escaping from one pressure chamber to the other pressure chamber.

The cover may include: a protrusion that fits in the interior space; and a receding surface positioned around the protrusion. The housing may include a top surface that is in contact with the receding surface. An inclined surface may be formed on an inner circumferential edge of the top surface, such that the seal groove is formed between the inclined surface and a corner between an outer circumferential surface of the protrusion and the receding surface. According to this configuration, in advance of attaching the cover to the housing, the outer sealing member can be disposed at the corner between the outer circumferential surface of the protrusion and the receding surface of the cover. This allows the cover to be readily attached to the housing.

For example, a proportion of a cross-sectional area of the outer sealing member to a cross-sectional area of the seal groove may be 90% or higher.

Robotic forceps according to one aspect of the present invention include: an insertion pipe; a gripper provided on a distal end of the insertion pipe, the gripper including a pair of tips facing each other, a first rotary actuator that swings one of the pair of tips, and a second rotary actuator that swings the other one of the pair of tips; and a third rotary actuator that swings the gripper relative to the insertion pipe. Each of the first rotary actuator, the second rotary actuator, and the third rotary actuator is the above-described rotary actuator. A rotational axis of the first rotary actuator and a rotational axis of the second rotary actuator are positioned coaxially. A rotational axis of the third rotary actuator is orthogonal to the rotational axis of the first rotary actuator and the rotational axis of the second rotary actuator.

Robotic forceps according to another aspect of the present invention include: an insertion pipe; a gripper provided on a distal end of the insertion pipe, the gripper including a pair of tips facing each other, a first rotary actuator that swings one of the pair of tips, and a second rotary actuator that swings the other one of the pair of tips; and a third rotary actuator that swings the gripper relative to the insertion pipe. A rotational axis of the first rotary actuator and a rotational axis of the second rotary actuator are positioned coaxially. A rotational axis of the third rotary actuator is orthogonal to the rotational axis of the first rotary actuator and the rotational axis of the second rotary actuator.

The above robotic forceps make it possible to arbitrarily change the orientation of the pair of tips, with which to grip an affected part of a patient, about the rotational axis of the third rotary actuator.

### Advantageous Effects of Invention

According to the present invention, the outer sealing member is interposed between the housing and the cover, and yet the working fluid can be hindered from escaping from one pressure chamber to the other pressure chamber, the one and the other pressure chambers being partitioned off from each other by the vane.

### Brief Description of Drawings

FIGS. 1A and 1B are perspective views each showing the distal end portion of robotic forceps, in which first to third rotary actuators each according to one embodiment of the present invention are incorporated; FIG. 1A shows tips being closed; and FIG. 1B shows the tips being opened.
FIG. 2 is a sectional view taken along line II-II of FIG. 1.
FIG. 3 is a sectional view taken along line III-III of FIG. 1.
FIG. 4 is a sectional view of a first rotary actuator.
FIG. 5 is an enlarged view of an essential part of FIG. 4.
FIG. 6 is a sectional view taken along line VI-VI of FIG. 4.
FIG. 7 is an enlarged sectional view of an essential part of the rotary actuator according to a variation.
FIG. 8 is a sectional view of the rotary actuator according to another variation.
FIG. 9 is a sectional view taken along line IX-IX of FIG. 8.

### Description of Embodiments

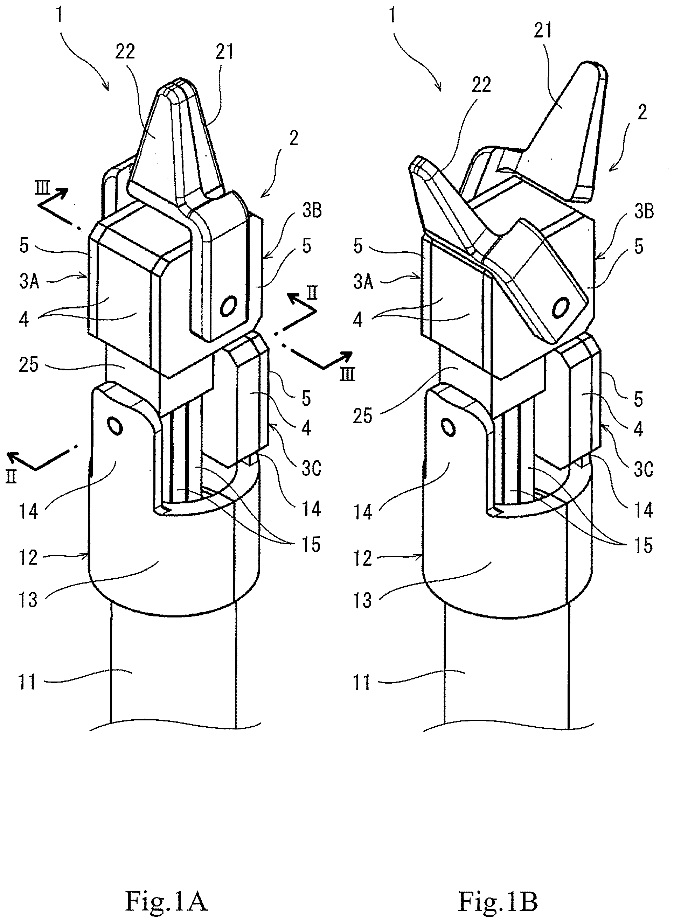

Each of FIGS. 1A and 1B shows the distal end portion of robotic forceps 1, in which first to third rotary actuators 3A to 3C each according to one embodiment of the present invention are incorporated.

The robotic forceps 1 are used in, for example, a surgery assisting system. In this case, the robotic forceps 1 are attached to a slave device, and a doctor operates the robotic forceps 1 by remote control using a master device.

Specifically, the robotic forceps 1 include: an insertion pipe 11 inserted in the body of a patient; and a gripper 2 provided on the distal end of the insertion pipe 11. The insertion pipe 11 may be a straight pipe having high stiffness, or may be a flexible pipe.

The gripper 2 includes: a pair of tips (a first tip 21 and a second tip 22) facing each other; the first rotary actuator 3A, which swings the first tip 21; and the second rotary actuator 3B, which swings the second tip 22. The robotic forceps 1 further include the third rotary actuator 3C, which swings the gripper 2 relative to the insertion pipe 11.

Each of the first to third rotary actuators 3Ato 3C is driven by a working fluid. In the present embodiment, the working fluid is a liquid, such as saline solution or oil. Although not illustrated, a drive unit is provided at the proximal end of the insertion pipe 11 (the opposite side to the gripper 2), and a supply/discharge device that supplies the working fluid to the first to third rotary actuators 3A to 3C and to which the working fluid from the first to third rotary actuators 3A to 3C is discharged is provided in the drive unit. Supplying of the working fluid from the unshown supply/discharge device to the first to third rotary actuators 3A to 3C, and discharging of the working fluid from the first to third rotary actuators 3Ato 3C to the supply/discharge device, are performed through a plurality of tubes 15, which are passed through the insertion pipe 11.

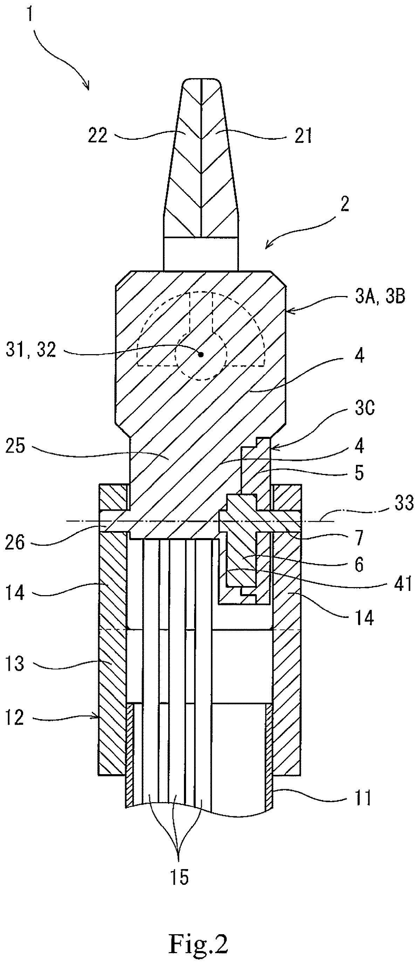

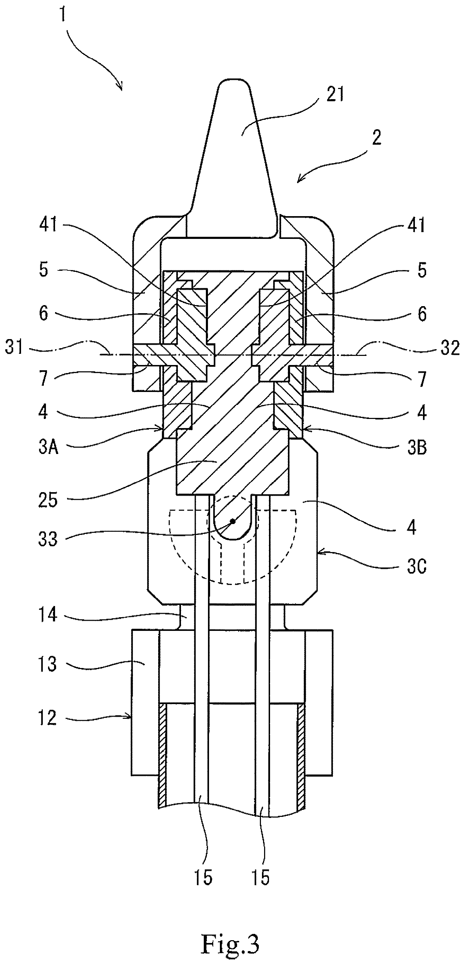

As shown in FIG. 3, a rotational axis 31 of the first rotary actuator 3A and a rotational axis 32 of the second rotary actuator 3B are positioned coaxially. A rotational axis 33 of the third rotary actuator 3C is orthogonal to the rotational axes 31 and 32 of the first rotary actuator 3A and the second rotary actuator 3B as shown in FIG. 2. Since the robotic forceps 1 are thus configured, the orientation of the first tip 21 and the second tip 22, with which to grip an affected part of a patient, can be arbitrarily changed about the rotational axis 33 of the third rotary actuator 3C.

It should be noted that, in the description below, for the sake of convenience of the description, the distal end side of the axial direction of the insertion pipe 11 is referred to as "upward", and the proximal end side of the axial direction of the insertion pipe 11 is referred to as "downward".

Hereinafter, a more specific description of the structure of the distal end portion of the robotic forceps 1 is given. A holding member 12 is fixed to the distal end of the insertion pipe 11. The holding member 12 is configured to be dividable into two half bodies. The holding member 12 includes a tubular part 13 and a pair of supporting pieces 14. The insertion pipe 11 is fitted to the inside of the tubular part 13. The pair of supporting pieces 14 protrudes upward from the tubular part 13, and the supporting pieces 14 face each other. Abase 25 of the gripper 2 and the third rotary actuator 3C are disposed between the pair of supporting pieces 14.

As shown in FIG. 2 and FIG. 3, each of the first to third rotary actuators 3A to 3C includes a housing 4 and a cover 5. The housing 4 includes an interior space 41, in which a vane 6 is disposed. The cover 5 is attached to the housing 4, and covers the interior space 41. In the present embodiment, the shape of each of the housing 4 and the cover 5 as seen in the axial direction of the rotary actuator (i.e., the direction in which the rotational axis of the rotary actuator extends) is substantially rectangular. Alternatively, the shape of each of the housing 4 and the cover 5 as seen in the axial direction of the rotary actuator may be a different shape, such as a circular shape.

In the present embodiment, the housing 4 of the first rotary actuator 3A, the housing 4 of the second rotary actuator 3B, the base 25, and the housing 4 of the third rotary actuator 3C are integrated together to form a single block. Alternatively, at least one of the housing 4 of the first rotary actuator 3A, the housing 4 of the second rotary actuator 3B, the base 25, and the housing 4 of the third rotary actuator 3C may be provided as a separate object.

In each of the first to third rotary actuators 3A to 3C, a rotational shaft 7 penetrating the cover 5 is integrally provided on the vane 6. The rotational shaft 7 of the first rotary actuator 3A is non-rotatably coupled to the first tip 21, and the rotational shaft 7 of the second rotary actuator 3B is non-rotatably coupled to the second tip 22. The rotational shaft 7 of the third rotary actuator 3C is non-rotatably coupled to one of the supporting pieces 14. That is, in the present embodiment, the housing 4 and the cover 5 of the third rotary actuator 3C rotate relative to the supporting pieces 14.

Alternatively, the positional relationship between the housing 4 and the cover 5 of the third rotary actuator 3C may be inverted from the positional relationship shown in FIG. 2; the housing 4 of the third rotary actuator 3C may be fixed to the supporting pieces 14; and the rotational shaft 7 may be non-rotatably coupled to the base 25. In this case, the shape of each of the housing 4 and the cover 5 as seen in the axial direction of the rotary actuator is, for example, a circular shape.

The base 25 is provided with a rotational shaft 26 coaxially with the rotational shaft 7 of the third rotary actuator 3C. The rotational shaft 26 is rotatably supported by the supporting piece 14 that is opposite to the supporting piece 14 to which the rotational shaft 7 of the third rotary actuator 3C is coupled.

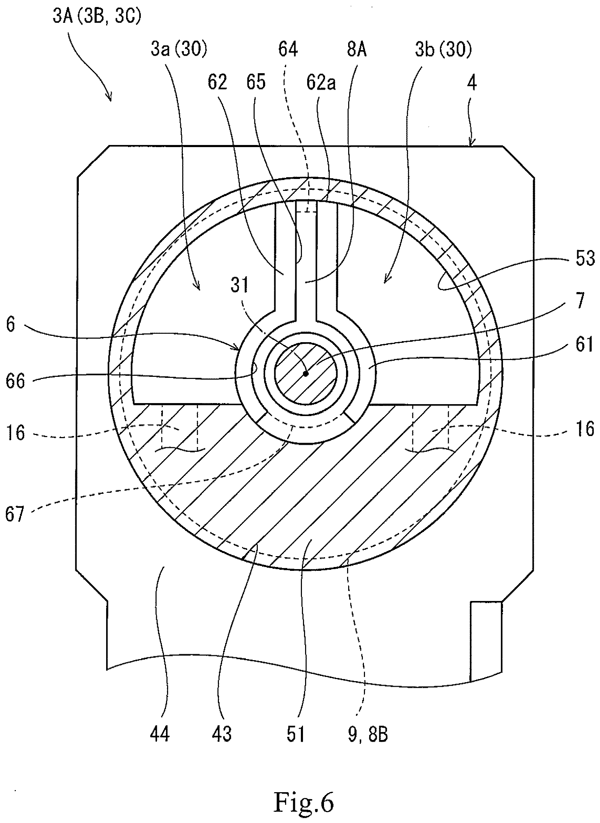

The aforementioned tubes 15 are connected to the base 25. Inside the block formed by the base 25 and the housings 4 of the first to third rotary actuators 3A to 3C, a plurality of passages 16 are formed extending from each tube 15 to the corresponding interior space 41 (see FIG. 6; the illustration is omitted in FIG. 2 and FIG. 3).

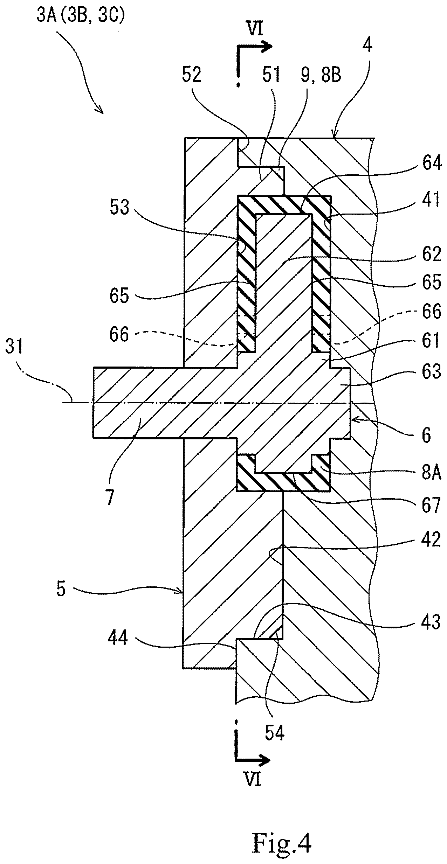

The first to third rotary actuators 3A to 3C have the same structure. Therefore, in the description below, the structure of the first rotary actuator 3A is described in detail as a representative example with reference to FIG. 4 to FIG. 6.

In the present embodiment, the shape of the interior space 41 of the housing 4 as seen in the axial direction of the first rotary actuator 3A may be a substantially semi-circular shape so that the vane 6 can swing within an angular range of 180 degrees. Alternatively, the shape of the interior space 41 of the housing 4 as seen in the axial direction of the first rotary actuator 3A may be a minor sector shape so that the vane 6 can swing within an angular range less than 180 degrees, or may be an incomplete circular shape (major sector shape) so that the vane 6 can swing within an angular range greater than 180 degrees.

In the present embodiment, the depth of the interior space 41 of the housing 4 is set to be about half of the height of the vane 6. However, the depth of the interior space 41 can be set arbitrarily, so long as the depth of the interior space 41 is less than the height of the vane 6.

The housing 4 includes: a reference surface 42 positioned around the interior space 41; an annular wall surface 43 rising from the outer circumferential edge of the reference surface 42; and a top surface 44 positioned around the wall surface 43. In the present embodiment, the outer contour of the reference surface 42 has a circular shape whose center is the rotational axis 31. Accordingly, the wall surface 43 has a circular cylindrical shape. Alternatively, the outer contour of the reference surface 42 may have such a substantially D shape that the interior space 41 has been enlarged.

The cover 5 includes: a protrusion 51, which fits inside the wall surface 43; and a receding surface 52 positioned around the protrusion 51. In the present embodiment, the distal end surface of the protrusion 51 is in contact with the reference surface 42 of the housing 4. The outer circumferential surface of the protrusion 51 faces the wall surface 43 of the housing 4, with a slight gap formed therebetween. The receding surface 52 faces the top surface 44 of the housing 4, with a slight gap formed therebetween.

A recess 53, which forms a vane-accommodating space 30 together with the interior space 41 of the housing 4, is formed in the protrusion 51. That is, the sum of the depth of the recess 53 and the depth of the interior space 41 is substantially equal to the height between the side surfaces of the vane 6. The vane 6 partitions off the vane-accommodating space 30 into a first pressure chamber 3a and a second pressure chamber 3b.

As described above, in the present embodiment, the distal end surface of the protrusion 51 of the cover 5 is in contact with the reference surface 42 of the housing 4. However, between the distal end surface of the protrusion 51 and the reference surface 42 of the housing 4, a leakage path of the working fluid from the vane-accommodating space 30 is formed due to, for example, the waviness and surface roughness of the distal end surface of the protrusion 51 and the reference surface 42 of the housing 4.

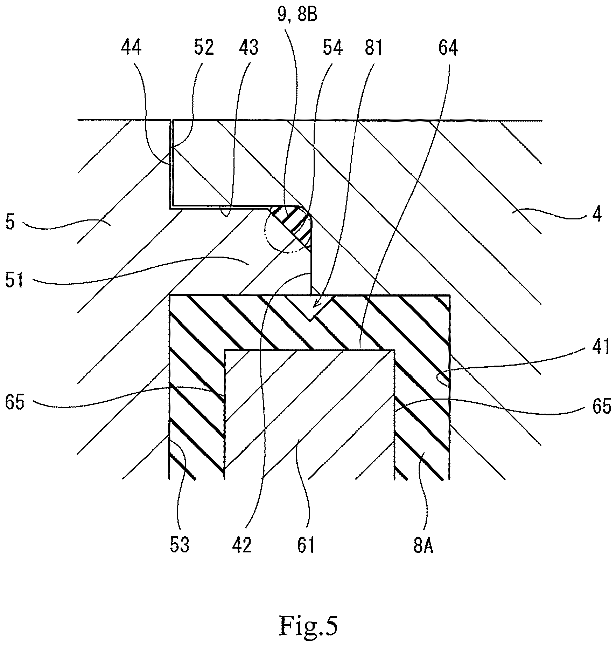

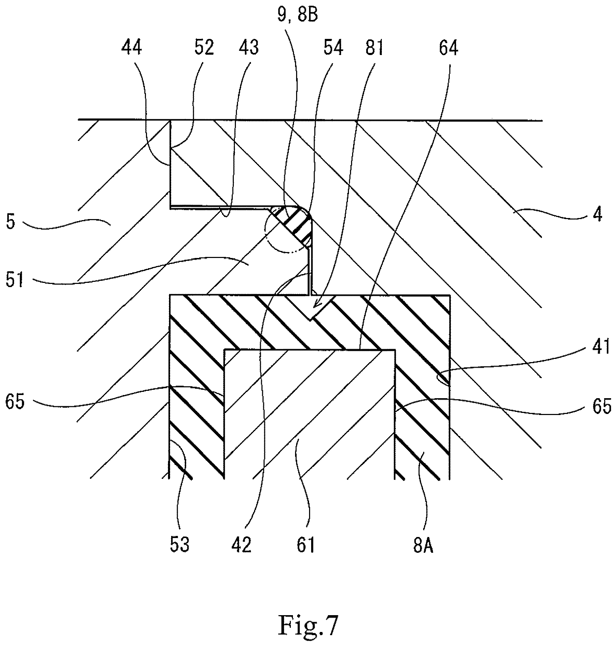

Alternatively, as shown in FIG. 7, the receding surface 52 of the cover 5 may be in contact with the top surface 44 of the housing 4, and the distal end surface of the protrusion 51 may face the reference surface 42, with a slight gap formed therebetween. However, if the structure shown in FIG. 5 is adopted, the amount of leakage from the vane-accommodating space 30 can be reduced compared to the structure shown in FIG. 7.

The vane 6 includes: a circular pillar 61 whose center is the rotational axis 31 of the first rotary actuator 3A; and a plate 62 protruding outward in the radial direction from the outer circumferential surface of the circular pillar 61. The above-described rotational shaft 7 protrudes from one end surface of the circular pillar 61, and is rotatably supported by the cover 5. The other end surface of the circular pillar 61 is provided with a shaft 63, and the shaft 63 is rotatably supported by the housing 4.

An inner sealing member 8A, which surrounds the plate 62 and the circular pillar 61, is attached to the vane 6. To be more specific, a straight groove 64, which extends in the axial direction of the circular pillar 61, is formed in a distal end surface 62a of the plate 62. One straight groove 65 extending in the radial direction of the circular pillar 61 is formed in the side surface of the plate 62 on the cover 5 side, and the other straight groove 65 extending in the radial direction of the circular pillar 61 is formed in the side surface of the plate 62 on the opposite side to the cover 5. Circular grooves 66 are formed in both end surfaces of the circular pillar 61, respectively. A wide-width groove 67 extending in the axial direction of the circular pillar 61 is formed in the outer circumferential surface of the circular pillar 61 at a position opposite to the plate 62. The inner sealing member 8A is inserted in these grooves 64 to 67.

A notch 81 is formed in a part of the inner sealing member 8A, the part being positioned on the distal end surface 62a of the plate 62 (to be exact, the part being inserted in the groove 64), at a position corresponding to the reference surface 42 of the housing 4 and the distal end surface of the protrusion 51. The notch 81 in the part of the inner sealing member 8A, the part being positioned on the distal end surface 62a of the plate 62, extends transversely in the thickness direction of the plate 62. In the illustrated example, the notch 81 has a triangular cross-sectional shape. Alternatively, the notch 81 may have, for example, a semicircular or rectangular cross-sectional shape.

An inclined surface 54 continuous in a circumferential direction is formed on the outer circumferential edge of the distal end surface of the protrusion 51 of the cover 5, such that an annular seal groove 9 having a triangular cross-sectional shape is formed between the inclined surface 54 and a corner between the reference surface 42 and the wall surface 43 of the housing 4. That is, the seal groove 9 is formed between the housing 4 and the cover 5 in a manner to surround the interior space 41.

An outer sealing member 8B is inserted in the seal groove 9. For example, as indicated by two-dot chain line in FIG. 5, the outer sealing member 8B is an O-ring having a circular cross-sectional shape when it is in a natural state. Alternatively, the cross-sectional shape of the outer sealing member 8B when it is in a natural state may be a different shape, such as an ellipsoidal shape.

The proportion of the cross-sectional area of the outer sealing member S2 to the cross-sectional area S1 of the seal groove 9 (S2/S1) is desirably 90% or higher, and more desirably 95% or higher. The cross-sectional area S1 of the seal groove 9 is the area of a section that is surrounded by: a line that is drawn by extending the inclined surface 54 to the reference surface 42 and to the wall surface 43; the reference surface 42; and the wall surface 43. The cross-sectional area of the outer sealing member S2 is the cross-sectional area of the outer sealing member when it is in a natural state.

As described above, in the first to third rotary actuators 3A to 3C of the present embodiment, since the seal groove 9 has a triangular cross-sectional shape, the filling ratio of the outer sealing member 8B in the seal groove 9 can be increased compared to a case where the seal groove 9 has a rectangular cross-sectional shape. In this manner, the outer sealing member 8B is interposed between the housing 4 and the cover 5, and yet the working fluid can be hindered from escaping from the first pressure chamber 3a to the second pressure chamber 3b or from the second pressure chamber 3b to the first pressure chamber 3a, the first and second pressure chambers 3a and 3b being partitioned off from each other by the vane 6.

Moreover, in the present embodiment, in advance of attaching the cover 5 to the housing 4, the outer sealing member 8B can be disposed at the corner between the reference surface 42 and the wall surface 43 of the housing 4. This allows the cover 5 to be readily attached to the housing 4.

Furthermore, in the present embodiment, the notch 81 is formed in the part of the inner sealing member 8A, the part being positioned on the distal end surface 62a of the plate 62. Therefore, when fitting the protrusion 51 of the cover 5 to the inside of the wall surface 43 of the housing 4, the inner sealing member 8A can be prevented from getting caught between the reference surface 42 of the housing 4 and the distal end surface of the protrusion 51.

### (Variations)

The present invention is not limited to the above-described embodiment. Various modifications can be made without departing from the scope of the present invention.

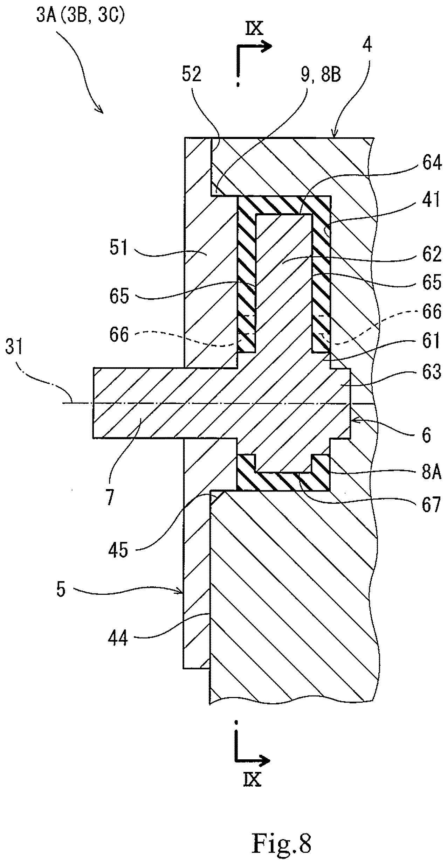

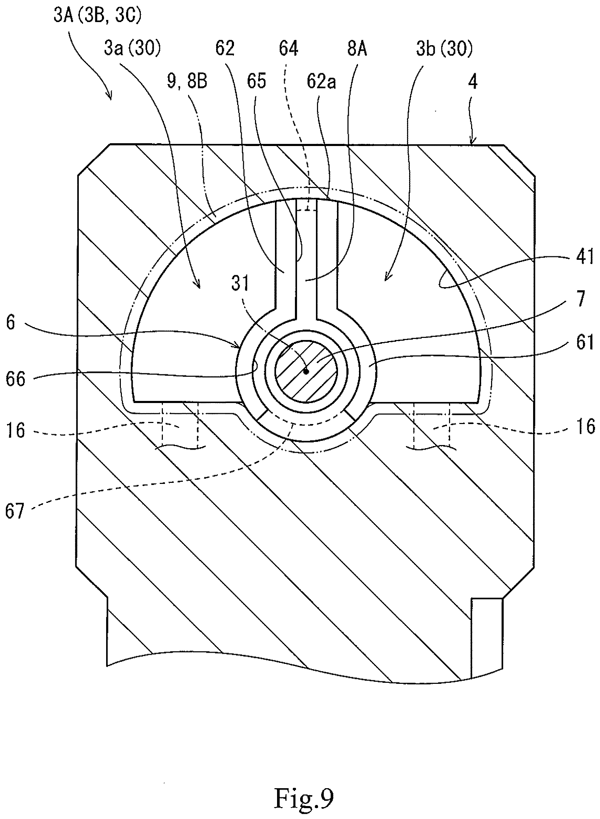

For example, as shown in FIG. 8 and FIG. 9, the depth of the interior space 41 of the housing 4 may be greater than the height of the vane 6, and the protrusion 51 of the cover 5 may be fitted in the interior space 41. In this case, the receding surface 52 of the cover 5 is in contact with the top surface 44 of the housing 4, and the outer circumferential surface of the protrusion 51 of the cover 5 faces the inner circumferential surface of the interior space 41, with a slight gap formed therebetween.

In the configurations shown in FIG. 8 and FIG. 9, an inclined surface 45 continuous in a circumferential direction is formed on the inner circumferential edge of the top surface 44 of the housing 4, such that an annular seal groove 9 having a triangular cross-sectional shape is formed between the inclined surface 45 and a corner between the outer circumferential surface of the protrusion 51 and the receding surface 52 of the cover 5. That is, the seal groove 9 is formed between the housing 4 and the cover 5 in a manner to surround the interior space 41.

According to the configuration shown in FIG. 8 and FIG. 9, similar to Embodiment 1, the filling ratio of the outer sealing member 8B in the seal groove 9 can be increased compared to a case where the seal groove 9 has a rectangular cross-sectional shape. In this manner, the outer sealing member 8B is interposed between the housing 4 and the cover 5, and yet the working fluid can be hindered from escaping from the first pressure chamber 3a to the second pressure chamber 3b or from the second pressure chamber 3b to the first pressure chamber 3a, the first and second pressure chambers 3a and 3b being partitioned off from each other by the vane 6.

Moreover, in the configuration shown in FIG. 8 and FIG. 9, in advance of attaching the cover 5 to the housing 4, the outer sealing member 8B can be disposed at the corner between the outer circumferential surface of the protrusion 51 and the receding surface 52 of the cover 5. This allows the cover 5 to be readily attached to the housing 4.

The seal groove 9 having a triangular cross-sectional shape need not be formed by utilizing a corner between a surface parallel to the radial direction of the rotary actuator and a surface orthogonal thereto. For example, the seal groove having a triangular cross-sectional shape may be formed by utilizing an annular recess that is recessed in a V shape from a surface parallel to the radial direction of the rotary actuator.

Further, in the above-described embodiment and the variation shown in FIG. 8 and FIG. 9, the interior space 41 of the housing 4 is open in one direction. Alternatively, the interior space 41 of the housing 4 may be open in one and the other directions, and the cover 5 may be disposed on both sides of the housing 4.

Although not illustrated, conversely to FIG. 8, the cover 5 may have a recess that is greater than the interior space 41 of the housing 4, and the housing 4 may be configured to fit in the recess of the cover 5.

Still further, the working fluid by which to drive the rotary actuator of the present invention need not be a liquid, but may be a gas. The rotary actuator of the present invention may be incorporated not only in robotic forceps, but also in other various equipment.

### Reference Signs List

- 1: robotic forceps
- 11: insertion pipe
- 2: gripper
- 21, 22: tip
- 3A: first rotary actuator
- 3B: second rotary actuator
- 3C: third rotary actuator
- 31 to 33: rotational axis
- 4: housing
- 41: interior space
- 42: reference surface
- 43: wall surface
- 44: top surface
- 5: cover
- 51: protrusion
- 52: receding surface
- 53: recess
- 54, 55: inclined surface
- 6: vane
- 61: circular pillar
- 62: plate
- 62a: distal end surface
- 8A: inner sealing member
- 8B: outer sealing member
- 81: notch
- 9: seal groove

## Claims

1. A rotary actuator comprising:
a housing including an interior space in which a vane is disposed; and
a cover that is attached to the housing and covers the interior space, wherein
an annular seal groove having a triangular cross-sectional shape is formed between the housing and the cover in a manner to surround the interior space, and an outer sealing member is inserted in the seal groove.

2. The rotary actuator according to claim 1, wherein
the housing includes:
a reference surface positioned around the interior space; and
an annular wall surface rising from an outer circumferential edge of the reference surface,
the cover includes a protrusion that fits inside the wall surface, and
an inclined surface is formed on an outer circumferential edge of a distal end surface of the protrusion, such that the seal groove is formed between the inclined surface and a corner between the reference surface and the wall surface.

3. The rotary actuator according to claim 2, wherein
a recess that forms a vane-accommodating space together with the interior space is formed in the protrusion.

4. The rotary actuator according to claim 3, wherein
the vane includes:
a circular pillar whose center is a rotational axis of the rotary actuator; and
a plate protruding outward in a radial direction from the circular pillar,
the rotary actuator further includes an inner sealing member attached to the vane, the inner sealing member surrounding the plate and the circular pillar, and
a notch is formed in a part of the inner sealing member, the part being positioned on a distal end surface of the plate, at a position corresponding to the reference surface of the housing and the distal end surface of the protrusion.

5. The rotary actuator according to any one of claims 2 to 4, wherein
the distal end surface of the protrusion is in contact with the reference surface of the housing.

6. The rotary actuator according to claim 1, wherein
the cover includes:
a protrusion that fits in the interior space; and
a receding surface positioned around the protrusion,
the housing includes a top surface that is in contact with the receding surface, and
an inclined surface is formed on an inner circumferential edge of the top surface, such that the seal groove is formed between the inclined surface and a corner between an outer circumferential surface of the protrusion and the receding surface.

7. The rotary actuator according to any one of claims 1 to 6, wherein
a proportion of a cross-sectional area of the outer sealing member to a cross-sectional area of the seal groove is 90% or higher.

8. Robotic forceps comprising:
an insertion pipe;
a gripper provided on a distal end of the insertion pipe, the gripper including a pair of tips facing each other, a first rotary actuator that swings one of the pair of tips, and a second rotary actuator that swings the other one of the pair of tips; and
a third rotary actuator that swings the gripper relative to the insertion pipe, wherein
each of the first rotary actuator, the second rotary actuator, and the third rotary actuator is the rotary actuator according to any one of claims 1 to 7,
a rotational axis of the first rotary actuator and a rotational axis of the second rotary actuator are positioned coaxially, and
a rotational axis of the third rotary actuator is orthogonal to the rotational axis of the first rotary actuator and the rotational axis of the second rotary actuator.

9. Robotic forceps comprising:
an insertion pipe;
a gripper provided on a distal end of the insertion pipe, the gripper including a pair of tips facing each other, a first rotary actuator that swings one of the pair of tips, and a second rotary actuator that swings the other one of the pair of tips; and
a third rotary actuator that swings the gripper relative to the insertion pipe, wherein
a rotational axis of the first rotary actuator and a rotational axis of the second rotary actuator are positioned coaxially, and
a rotational axis of the third rotary actuator is orthogonal to the rotational axis of the first rotary actuator and the rotational axis of the second rotary actuator.
